# EUROPEAN PATENT SPECIFICATION

(11) **EP 2 105 053 B1**
(45) Date of publication and mention of the grant of the patent: **25.07.2012**
(21) Application number: 07848125.6
(22) Date of filing: 12.11.2007
(51) Int. Cl.: A22C 25/04, A01K 61/00

(54) **METHOD AND EQUIPMENT FOR DETERMINING THE SEX OF FISH**
VERFAHREN UND AUSRÜSTUNG ZUR BESTIMMUNG DES GESCHLECHTS VON FISCHEN
MÉTHODE ET ÉQUIPEMENT POUR DÉTERMINER LE SEXE DES POISSONS

(43) Date of publication of application: 30.09.2009
(73) Proprietor: Fundacion Azti-azti Fundazioa, 48395 Vizcaya (ES)
(72) Inventor: MARTINEZ DE MARAÑÓN IBABE, Iñigo, 48395 Sukarrieta (ES); RODRIGUEZ FERNÁNDEZ, Raquel, 48395 Sukarrieta (ES); DUCH ORLEANS, Antonio I., 48395 Sukarrieta (ES)
(74) Representative: Ezcurra Zufia, Maria Antonia
(86) International application number: PCT/ES2007/070182
(87) International publication number: WO 2009/063101

(56) References cited:
- CN-A- 1 601 275
- US-A- 4 051 952
- US-A- 5 013 906

## Description

### OBJECT OF THE INVENTION

The present method refers to a method and the equipment for determining the sex of fish, in particular fish which, due to their anatomy, cannot be sexed by non-invasive means.

The method which is the subject of this invention enables the fish's sex to be determined by measuring the colour of the gonads.

### BACKGROUND TO THE INVENTION

Various methods are known for sexing fish.

These methods may be categorised as invasive, minimally invasive, and non-invasive.

Among the technologies concerned with fish sexing we may find rudimentary methods based on the application of pressure to the abdomen of the fish and subsequent observation of the fluid emanating from the anus. These methods, whilst simple, have the disadvantage of the amount of time needed, which implies additional costs in the procedure. They also produce alterations in the roe extracted, due to the pressure exercised on the fish, and thus a loss of quality is also involved.

Other, more developed, technologies which are currently in use to date are those known as non-invasive methods. The sexing of different species of fish has been described through analysis of results obtained from external electromagnetic irradiation, both manually and automatically, however, these methods are only applicable to certain species of fish with well defined external morphological characteristics. The present invention shows a method devised for species which, due to their morphological characteristics do not permit sexing by electromagnetic irradiation in the external body of the fish, but which require irradiation directly in or on the gonads of the fish to be sexed.

Document US4051952 describes a sensor for sexing fish by means of detecting the amount of energy transmitted through the fish, so that in the female the amount of light reaching the signal receiver is greater than in the male fish. This is due to the fact that the female fish is more transparent than the male. This method is only successful in species of fish where the transmission of light differs between male and female, and in species which do not have a thick body such as the herring, so that light is able to pass through the fish. In species where such differences do not exist, the method is not effective and therefore the methodology developed in this invention has been proposed.

Document US3859522 refers to a system for sexing fish by means of an amount of light received following irradiation in the gonad region, with this amount of light being greater in the case of females. The method described in the invention is invasive as opposed to that described in this patent, and the analysis is made directly on the gonads, not solely in the gonad region

It is also known from the state of the art a sex determination method as the one disclosed in CN 1601275, which involves placing fish in to electric anesthetization tank, adopting low-voltage direct current, inserting endoscope into genital aperture and making dynamic determination to obtain determinable data

The present invention is extremely useful for species of fish which are of interest in Europe, such as the common mackerel, as sex is determined through the development of a sensor system which includes a measurement probe, a cleaning system and mechanical protection.

### DESCRIPTION OF THE INVENTION

Determining the sex of a fish is important, due to the added value of the roe in respect of the whole fish.

The present invention solves this problem of sexing those species of fish which, due to their morphological and/or physical and chemical characteristics cannot be sexed by non-invasive methods.

The solution has been to develop a method which is as non-invasive as possible, in order to determine the sex of these fish species.

Therefore, an initial aspect of this invention refers to a method for sexing fish based on the difference in colour between the gonads of the male and female according to claim 1, and consists of inserting a probe in or onto the fish's gonads and analysing the spectrum obtained following electromagnetic radiation in or on the gonad.

In order to obtain differentiating data in the range of visible spectrum, the light in or on the gonads, and its take up through reflection are transferred through a probe.

The probe may form part of a sensory system which, in addition to the probe which transmits the light, comprises a casing which provides resistance and prevents breakage of the probe, and facilitates the introduction of the entire system into the body of the fish to be sexed, as well as a cleaning system required to prevent and /or remove dirt on the point of the probe thus enabling data on the colour of the gonads to be obtained. For correct functioning of the method or sensory system, optimum positioning of the probe in the casing interior is necessary. The position of the probe inside the casing is crucial in order to avoid any interference in the measurement.

In order to introduce the sensory system in or on the gonads, there are various methods, one of which consists of passing the sensory system directly through the fish's flesh, another involves placing the sensory system on the surface of the fish and introducing it by means, for example, of a pneumatic system, until the desired area is reached and finally, the sensory system could be maintained in a fixed position and the fish would be moved until the probe reaches the optimum measurement zone.

In order to avoid damage to the probe it may be protected by a casing. There are two systems and procedures which ensure that the probe is situated in an optimum zone. The first system in which the probe and the casing are fixed in respect to each other, and the second in which the elements are mobile in respect of each other. In the first case the procedure consists of accessing the gonad as a group until the probe reaches the optimum position for measurement, whereas in the second method the system may function in the following manners; once the sensory system is inside the fish, the probe may move and the casing is fixed, or the casing may be mobile and the probe fixed, in these cases the corresponding part will be moved to ensure that the probe reaches the optimum measurement zone.

The casing may be made from plastic, glass or metal material. The casing, in addition to providing mechanical protection, may also incorporate a cleaning system for the sensory system or the probe-casing arrangement.

The cleaning system may be by means of air or water, and in turn the cleaning should take place between the channel made between the probe and the casing and/or the external part of the casing in a direction transversal to the probe. The internal cleaning system may be applied inside the fish or out, that is, between measurements of two fish. The cleaning system on the outside of the casing may be applied either inside or outside the fish, however this type of cleaning is recommended only when the sensory system is on the outside the fish so as not to disturb the quality of the sexed fish.

The probe may be made of a light tube or optic fibre or an equivalent device which enables light to be transferred from the source to the gonads and from these to the detector.

A probe without a casing may be used for the sexing process, although it is recommended that it be used to provide rigidity and prevent breakage of the probe after various applications.

The method proposed for detection is applicable both for manual or automatic differentiation of the fish species of interest.

This method permits the fish to be classified in a very short time, less than 5 seconds, and since the method involves minimal invasion, at the end of the process the treated sample has a perforation in the abdomen of some 2mm in diameter, and therefore the fish's gonads and the whole fish remains practically in its original state prior to sexing, and thus both may be made use of.

### DESCRIPTION OF THE DRAWINGS

The present descriptive report is complemented by a set of plans illustrating preferred embodiments of the invention but which is in no way restrictive.
Figure 1 shows a diagram of the equipment used for sexing.
Figure 2 shows a section of the sensory system using an example of an embodiment.
Figure 3 shows three positions of the probe in the casing.
Figure 4 shows a section of an example of pneumatic impulsion of the sensory system by means of a gun.
Figure 5 shows a section of a possible casing design.

### PREFERRED EMBODIMENT OF THE INVENTION

### First example of an embodiment.

The fish, (1) in this case of an embodiment using a mackerel, is presented to the operator by manual feeding, the mode of use in this method refers to manual application.

The sensory system (2) is introduced into the fish's gonad and having subjected the sample to electro-magnetic radiation with a wavelength range between 360 and 2000 mm, the spectrum obtained in a visible range (360-760 mm)processing it in an appropriate manner in order to generate a digital signal, for the purpose of classifying the fish as male or female.

The measurement probe comprises 7 200 um optic fibres, 6 of which are for illumination purposes and one for reading. The fibres are covered by a plastic material with the exception of the end part which is introduced into the mackerel, and which is covered with stainless steel tip measuring 1.5 mm diameter and 50mm. long.

The probe is covered by a metal casing with a bevelled point to provide it with sufficient resistance so that it does not break.

The perforation made in the mackerel is 2mm in diameter and does not in any way prejudice, either in appearance or quality, the fish or its gonads.

In the invention procedure, data is obtained based on the sensory system (2) which permits, once the signal treatment has been applied, characterisation of the electromagnetic spectrum of the wave reflected with respect to the incidence of the colour of the gonads.

A rapid response of less than five seconds is obtained.

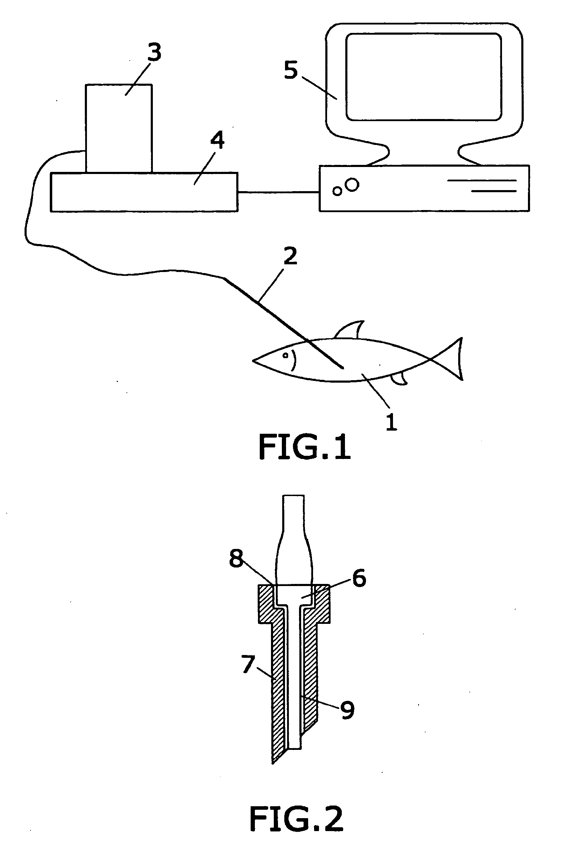

Figure 1 shows a diagram of the equipment used. The equipment includes a spectrometer (3) which is provided with a photodiode, a source of energy (4), a sensory system (2) a computer (5). The equipment is also provided with a spectrometer data collection programme which also transforms the data in order to classify the fish

The source of energy (4) generates and transmits in a range of wavelengths between 360-2000 mn.

The light energy is transmitted from the source (4) to the sample (1) through the probe. The energy collected is converted into voltage through an A/D converter (analogue/digital) to be analysed in a micro-processor.

In order to ensure correct collection of the data the following steps should be taken:

Presentation of the fish (1) to the operator and presentation of this to the sensory system (2), determination of the optimum zone for perforating the fish depending on the size, whether the perforation is made in or on the gonad, cleaning the sensory system, trans-mission and measurement of the incident and reflected light, calculation of the parameters, extraction of differentiating parameters, classification of fish in terms of their sex. Prior to commencing the classification the procedure is calibrated.

Figure 2 shows the probe (6) protected by a casing (7) used in this example of an embodiment. Cleaning of the probe (6) arrangement and the casing (7) is made through small channels and on the external part using compressed air. There are channels (9) between the casing (7) and the probe (6) for the passage of cleaning fluid.

As shown in figure 2, the casing (7) is bevelled at the end which is inserted into the fish, in order to facilitate the perforation.

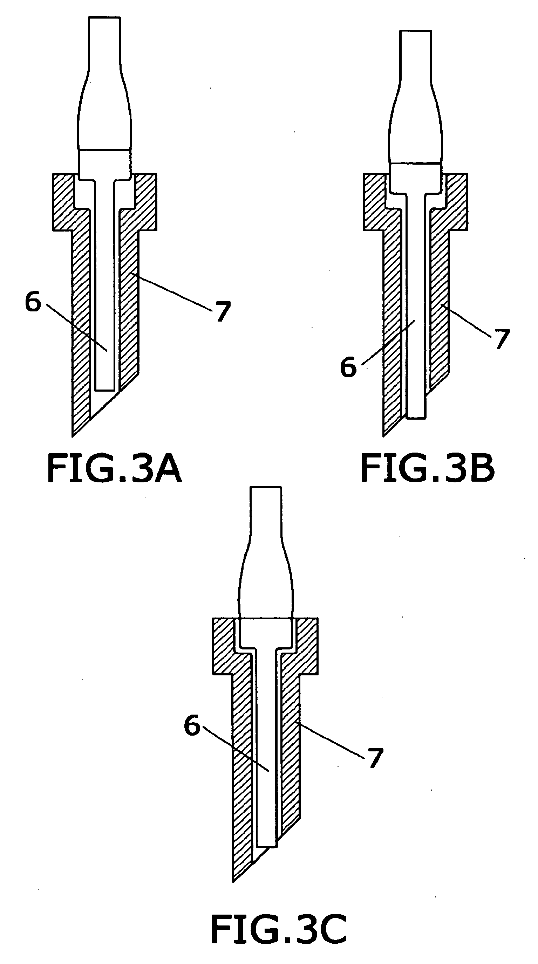

It is important for the probe (6), as shown in figure 3c, to project beyond the casing (7) so that the recording can be taken without any interference from the casing (7). Figure 3 shows an arrangement of the probe (6) in the interior of the casing, taking into account the fact that, if the end of the casing is too high as in fig. 3a, the probe will measure the interior of the casing, and conversely, if the probe is too low as in fig. 3b there is a risk of breaking the probe. The optional position is shown in figure 3c.

The sensory system (2) in this embodiment is introduced directly into the fish.

### Second example of an embodiment

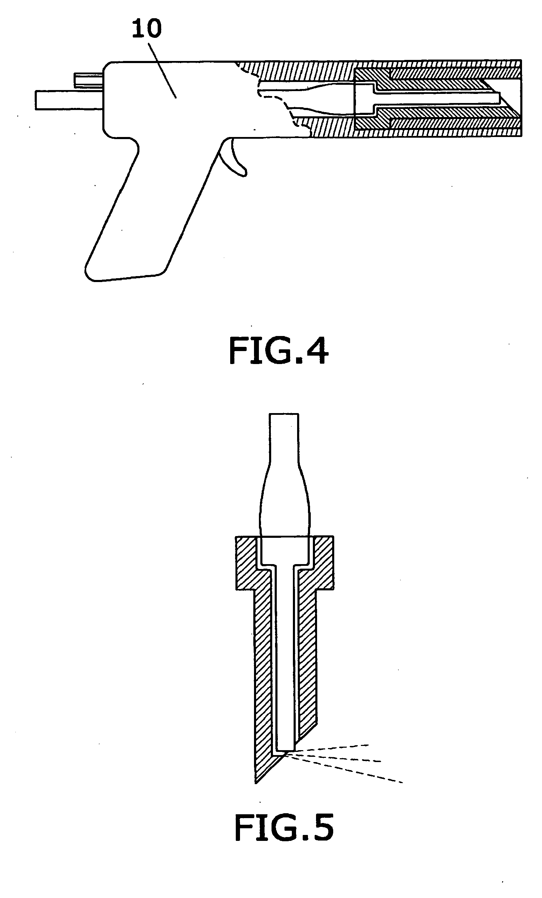

The sensory system (2) in this embodiment could be placed on the surface of the fish and introduced, for example by means of a pneumatic system until the desired zone is reached. In this example of an embodiment, the pneumatic system is a gun (10) as shown in figure 4.

### Third example of an embodiment

The casing of the sensory system, in this embodiment, as shown in figure 5 could be made by circulating a fluid in longitudinal direction to this and bearing in mind that at the end of the route round the interior of the casing, the direction of the fluid changes, remaining in direction towards the point of the measurement probe.

The essential nature of this invention is not altered by any variations in materials, form, size and arrangement of its component elements, which are described in a non-restrictive manner, with this being sufficient to proceed to its reproduction by an expert.

## Claims

1. Method for determining the sex of a fish based on the difference in colour between the gonads of the male and the female, which consists of inserting a probe (6) in or on the fish gonad and analysing the spectrum obtained following the incidence of an electromagnetic radiation on the gonad.

2. Method according to claim 1 **characterised in that** the probe (6) forms part of a sensory system which includes, in addition to the measurement probe (6) a casing (7) and a cleaning system.

3. Method according to claim 1 **characterised in that** it includes the following steps: Presentation of the fish (1) to the operator or processing zone, and presentation of this to the sensory system (2), determination of the optimum zone for perforating the fish depending on its size, and the situation of the gonads in the species in question, cleaning the sensory system, transmission and measurement of the incident and reflected light, calculation of the parameters, extraction of differentiating parameters, classification of fish in terms of their sex.

4. Method according to claim 1 **characterised in that** the spectrum obtained is situated within the visible range.

5. Method according to claim 1 **characterised in that** the fish is mackerel.

6. Method according to claim 1 **characterised in that** the probe (6) is made from optic fibre.

7. Equipment for carrying out the method described in the previous claims, **characterised in that** it includes: a spectrometer (3) which is provided with a photodiode, a source of energy (4), a sensory system (2) and a computer (5).

## Patentansprüche

1. Methode zur Bestimmung des Geschlechts eines Fisches aufgrund des Farbunterschieds zwischen den Gonaden des Männchens und des Weibchens, die in der Einführung einer Sonde (6) in oder auf die Fischgonade und der Untersuchung des durch Einfall einer elektrischen Strahlung auf die Gonade erzielten Spektrums besteht.

2. Methode gemäß Patentanspruch 1, **dadurch gekennzeichnet, dass** die Sonde (6) Teil einer Sensorik ist, die neben der Messsonde (6) ein Gehäuse (7) und ein Reinigungssystem umfasst.

3. Methode gemäß Patentanspruch 1, **dadurch gekennzeichnet, dass** sie folgende Schritte umfasst: Präsentation des Fisches (1) an den Arbeiter oder Verarbeitungsbereich und dessen Präsentation an die Sensorik (2), Bestimmung des optimalen Bereichs zur Durchbohrung des Fisches je nach seiner Größe und der Lage der Gonaden bei der fraglichen Spezies, Reinigung der Sensorik, Übertragung und Messung des einfallenden und reflektierten Lichts, Berechnung der Parameter, Ermittlung der unterscheidenden Parameter und Einstufung des Fisches nach seinem Geschlecht.

4. Methode gemäß Patentanspruch 1, **dadurch gekennzeichnet, dass** das erzielte Spektrum im sichtbaren Bereich liegt.

5. Methode gemäß Patentanspruch 1, **dadurch gekennzeichnet, dass** der Fisch eine Makrele ist.

6. Methode gemäß Patentanspruch 1, **dadurch gekennzeichnet, dass** die Sonde (6) aus Glasfaser besteht.

7. Ausrüstung zur Ausführung der in den vorigen Ansprüchen beschriebenen Methode, **dadurch gekennzeichnet, dass** sie Folgendes aufweist: einen Spektrometer (3), der mit einer Leuchtdiode ausgestattet ist, eine Energiequelle (4), eine Sensorik (2) und einen Rechner (5).

## Revendications

1. Méthode pour la détermination du sexe d'un poisson basé sur la différence de couleur entre les gonades du mâle et de la femelle, qui consiste en l'insertion d'une preuve (6) dans ou sur la gonade du poisson et en analysant le spectre obtenu à la suite de l'incidence d'une radiation électromagnétique sur la gonade.

2. Méthode selon la revendication no. 1 **caractérisée par le fait que** la preuve (6) fait partie d'un système sensoriel qui inclut, en plus de la preuve de mesure (6) un système type carcasse (7) et un système de nettoyage.

3. Méthode selon la revendication no. 1 **caractérisée par le fait qu'**il inclut les pas suivants : Présentation du poisson (1) à l'opérateur ou à la zone de procession, et la présentation de celui-ci au système sensoriel (2), détermination de la zone optimale pour la perforation du poisson en fonction de sa taille et la situation des gonades pour les espèces en question, le nettoyage du système sensoriel, la transmission et la mesure de l'incidence et de la lumière réfléchie, le calcul des paramètres, l'extraction des paramètres de différenciation, la classification du poisson en terme de leur sexe.

4. Méthode selon la revendication no. 1 **caractérisée par le fait que** le spectre obtenu est situé à l'intérieur du champ visible.

5. Méthode selon la revendication no. 1 **caractérisée par le fait que** le poisson est maquereau.

6. Méthode selon la revendication no. 1 **caractérisée par le fait que** la preuve (6) est fabriqué en fibre optique.

7. Equipement pour la mise en oeuvre de la méthode décrite dans les revendications précédentes, **caractérisé par le fait qu'**il inclut : un spectromètre (3) qui est prévu d'une photodiode, une source d'énergie (4), un système sensoriel (2) et un ordinateur (5).
